(19)

Europäisches
Patentamt
European
Patent Office
Office européen
des brevets

(11) **EP 4 435 791 A1**

(12) **EUROPEAN PATENT APPLICATION**
published in accordance with Art. 153(4) EPC

(43) Date of publication:
**25.09.2024 Bulletin 2024/39**

(21) Application number: **21964431.7**

(22) Date of filing: **19.11.2021**

(51) International Patent Classification (IPC):
*G16B 20/20* (2019.01)

(52) Cooperative Patent Classification (CPC):
**G16B 20/20**

(86) International application number:
**PCT/CN2021/131904**

(87) International publication number:
**WO 2023/087277 (25.05.2023 Gazette 2023/21)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **BGI Genomics Co., Limited
Guangdong 518083 (CN)**

(72) Inventors:
- **XIONG, Yun**
  **Shenzhen, Guangdong 518083 (CN)**
- **LIU, Shuixia**
  **Shenzhen, Guangdong 518083 (CN)**
- **ZENG, Quanlei**
  **Shenzhen, Guangdong 518083 (CN)**
- **ZHANG, Yu**
  **Shenzhen, Guangdong 518083 (CN)**

- **WEI, Yi**
  **Shenzhen, Guangdong 518083 (CN)**
- **LI, Hong**
  **Shenzhen, Guangdong 518083 (CN)**
- **FANG, Ting**
  **Shenzhen, Guangdong 518083 (CN)**
- **CHAI, Xianghua**
  **Shenzhen, Guangdong 518083 (CN)**
- **WANG, Mengjie**
  **Shenzhen, Guangdong 518083 (CN)**
- **YUAN, Yuying**
  **Shenzhen, Guangdong 518083 (CN)**
- **LI, Ning**
  **Shenzhen, Guangdong 518083 (CN)**

(74) Representative: **Winter, Brandl - Partnerschaft
mbB
Alois-Steinecker-Straße 22
85354 Freising (DE)**

(54) **SEQUENCE VARIATION ANALYSIS METHOD AND SYSTEM, AND STORAGE MEDIUM**

(57)    Provided are a sequence variation analysis method and system, and a storage medium, relating to the technical field of gene detection. The sequence variation analysis method includes: acquiring sequence variation data to be analyzed; obtaining a first variation feature set and a second variation feature set by performing feature extraction on the sequence variation data to be analyzed; inputting the first variation feature set into a trained first phenotype relationship prediction model, to obtain a first phenotype relationship prediction result, and inputting the second variation feature set into a trained second phenotype relationship prediction model, to obtain a second phenotype relationship prediction result; and obtaining a third phenotype relationship prediction result by taking a union of the first phenotype relationship prediction result and the second phenotype relationship prediction result.

| |
|---|
| Acquiring sequence variation data to be analyzed — S1 |
| Obtaining a first variation feature set and a second variation feature set by performing feature extraction on the sequence variation data to be analyzed — S2 |
| Inputting the first variation feature set into a trained first phenotype relationship prediction model, to obtain a first phenotype relationship prediction result, and inputting the second variation feature set into a trained second phenotype relationship prediction model, to obtain a second phenotype relationship prediction result — S3 |
| Obtaining a third phenotype relationship prediction result by taking a union of the first phenotype relationship prediction result and the second phenotype relationship prediction result — S4 |

FIG. 1

Processed by Luminess, 75001 PARIS (FR)

**Description**

**FIELD**

The present disclosure relates to the technical field of gene detection, and in particular, to a sequence variation analysis method and system, and storage medium.

**BACKGROUND**

Genetic defects are the leading cause of early miscarriage, stillbirth, perinatal deaths, infant deaths, and congenital disability. With the application and popularization of second generation sequencing technology, people are paying more attention to the prevention and control of birth defects, and it is possible to quickly, accurately, and inexpensively screen or diagnose a variety of diseases at once. However, it is an urgent and important issue to be optimized to interpret Next Generation Sequencing (NGS) sequencing results objectively, accurately, and efficiently, for guiding clinical application.

Gene detection procedures generally include sample processing, gene sequencing, variation identification, variation annotation, variation interpretation, variation verification, and detection reports. At present, gene interpretation mainly has the following problems.

1) The interpreting of pathogenicity of gene variation based on gene detection data rely on that professionals manually search various databases and literature, which usually takes a lot of manpower and time. In addition, gene interpretation has high requirements for practitioners, requiring practitioners to have knowledge reserves on genes, variations, diseases, etc., and since interpretation is based on experience logic of practitioners, interpretation may have different results from person to person.

2) Most variation annotation tools cannot identify complex variations, resulting in incorrect annotation information, which may further lead to misinterpretation. For example, the interpretation of the clinical significance of variation usually adopts the ranking technique of common variation types (such as SNV/InDel) and pathogenicity machine learning model, which mainly employs public database/software data information to perform machine learning training or combines certain artificial experience logic to obtain the ranking and classification algorithm of the clinical significance of variation. However, since the information source is not comprehensive enough and limited to volume of training, the accuracy of such a technique cannot be guaranteed, and the interpretation results of partial variation may be missing.

3) For the data obtained through detection, the results obtained based on manual or automated interpretation may include manual subjective differences or misinterpretation, and thus it is necessary to review the results. Due to the lack of rapid and accurate location of the variation to be reviewed, the review workload is huge, resulting in unnecessary labor costs.

4) Due to the uniformity of NGS sequencing technology, some gene variation detection results are required to subject to further experimental verification. Due to the lack of effective quality classification standards for variation sites, the verification of detection results mainly depends on manual subjective judgment. For the interpretation of pathogenic variation, variation verification is required to ensure the accuracy of interpretation results. A common variation verification includes manually performing primer design, PCR (Polymerase Chain Reaction) amplification, first generation sequencing, and result analysis for the segment where the variation site is located, which consumes a lot of manpower, time, and detection costs.

**SUMMARY**

The present disclosure provides a sequence variation analysis method and system, and a storage medium, for quickly locating the variation to be reviewed, thereby reducing the labor cost and enhancing the efficiency of review and accuracy of interpretation of the gene detection report.

In a first aspect, the present disclosure provides a sequence variation analysis method including: acquiring sequence variation data to be analyzed; obtaining a first variation feature set and a second variation feature set by performing feature extraction on the sequence variation data to be analyzed; inputting the first variation feature set into a trained first phenotype relationship prediction model, to obtain a first phenotype relationship prediction result, and inputting the second variation feature set into a trained second phenotype relationship prediction model, to obtain a second phenotype relationship prediction result; and obtaining a third phenotype relationship prediction result by taking a union of the first phenotype relationship prediction result and the second phenotype relationship prediction result.

In a second aspect, the present disclosure provides a computer-readable storage medium having a computer program stored thereon. The computer program, when being executed by a processor, implements the sequence variation analysis method described above.

In a third aspect, the present disclosure provides a sequence variation analysis system including: an acquisition module configured to acquire sequence variation data to be analyzed, and a first analysis module. The first analysis module is configured to: obtain a first variation feature set and a second variation feature set by performing feature extraction on the sequence variation data to be analyzed; input the first variation feature set into a trained first phenotype relationship prediction model, to obtain a first phenotype relationship prediction result, and input the second variation feature set into a trained second phenotype relationship prediction model, to obtain a second phenotype relationship prediction result; and obtain a third phenotype relationship prediction result by taking a union of the first phenotype relationship prediction result and the second phenotype relationship prediction result.

## BRIEF DESCRIPTION OF THE DRAWINGS

Other features, objects, and advantages of the present disclosure will become more apparent upon reading the detailed description of non-limiting embodiments with reference to the following drawings.

FIG. 1 is a flow chart of a sequence variation analysis method according to a first embodiment of the present disclosure;

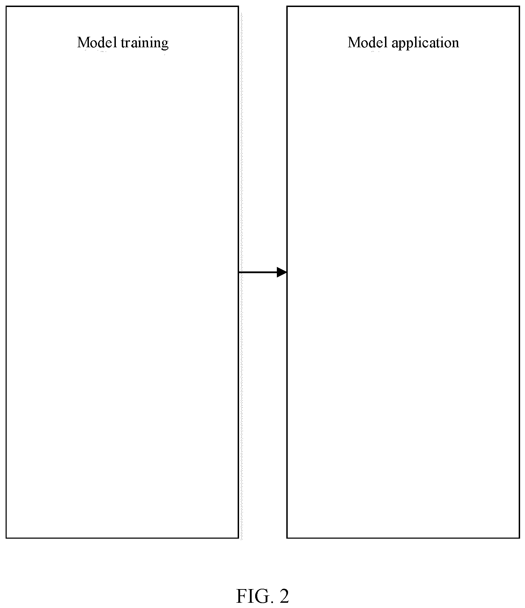

FIG. 2 is a schematic diagram illustrating a structure and application of a prediction model according to an embodiment of the present disclosure;
FIG. 3 is a flow chart of a sequence variation analysis method according to a second embodiment of the present disclosure;
FIG. 4 is a flow chart of a sequence variation analysis method according to a third embodiment of the present disclosure;
FIG. 5 is a flow chart of a sequence variation analysis method according to a fourth embodiment of the present disclosure;
FIG. 6 is a schematic diagram illustrating variation type prediction analysis result according to an example of the present disclosure;
FIG. 7 is a schematic diagram illustrating phenotype relationship prediction result according to an example of the present disclosure; and
FIG. 8 is a structural block diagram of a sequence variation analysis system according to an embodiment of the present disclosure.

## DETAILED DESCRIPTION

In the present disclosure, technical aspects, and advantages are described in detail below in conjunction with the accompanying drawings and embodiments. It should be understood that the specific embodiments described herein are illustrative only and are not limited to the present disclosure. In order to illustrate aspects of the present disclosure, specific embodiments are set forth below.

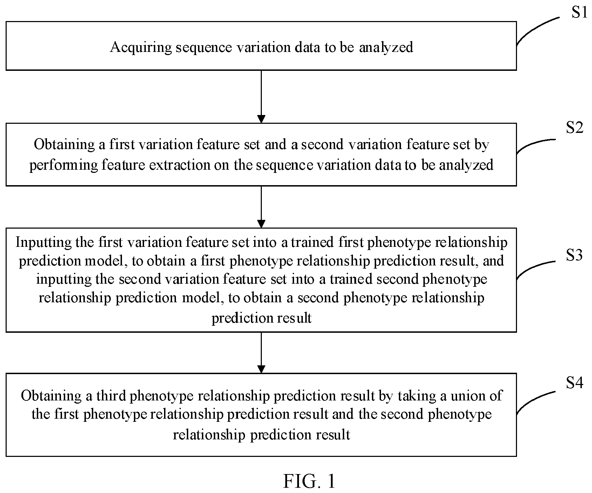

FIG. 1 is a flow chart of a sequence variation analysis method according to a first embodiment of the present disclosure.

As illustrated in FIG. 1, the sequence variation analysis method includes the following steps:

S 1, sequence variation data to be analyzed are acquired.

In an embodiment of the present disclosure, to facilitate analysis, data searching and processing are performed using the unique identifier of each variation in the sequence variation data to be analyzed. The unique identifier has a certain and unique mapping relationship with the variation. For example, the common "chromosome number-genome coordinates-reference sequence-alter sequence" (chr-pos-ref-alt, short for cpra) in this field is used as the unique variation identifier, where chr is the chromosome number, pos is the physical location of the reference sequence, ref is the reference sequence, and alt is the variation sequence.

The sequence variation data to be analyzed includes at least one variation site information. The variation site information includes at least the variation site and a unique identifier of the variation site. Specifically, the variation site information can be obtained by biological information analysis, which may include various conventional steps, such as quality control, filtration, alignment, etc. The technical means for acquiring the data are not limited, for example, specific sequence information can be obtained by various sequencing techniques.

S2, a first variation feature set and a second variation feature set are obtained by performing feature extraction on the sequence variation data to be analyzed.

In a preferred embodiment, each first variation feature value in the first variation feature set is a published phenotype relationship determination result of a variation site, and each second variation feature value in the second variation feature set is allele frequency data of the variation site and functional prediction data.

Specifically, when the present solution is applied to the analysis of human Mendelian genetic disease-related variation, the aforementioned phenotype relationship refers to the relationship between variation and clinical phenotype, and more specifically, to the pathogenicity of variation.

The first variation feature value can be obtained from the included information of various public databases, such as HGMD (http://www.hgmd.cf.ac.uk/ac/index.php), ClinVar (https://www.ncbi.nlm.nih.gov/clinvar/), LOVD (https://www.lovd.nl/3.0/search), etc. In the present disclosure, the source of the first variation feature value, a specific database and the number of databases are not limited. The phenotype relationship determination results recorded in various databases are usually based on personalized determination rules, and the determination results thereof are often described in personalized fields. As an example, for HGMD, the phenotype relationship determination results include disease-causing mutation (DM), likely disease-causing mutation (DM?), disease-associated polymorphism (DP), in vitro or in vivo functional polymorphism (FP), disease-associated polymorphism with additional functional evidence (DFP), and retired record (R). In an embodiment of the present disclosure, a first variation feature may be extracted from a public database based on the unique identifier (e.g., cpra) of each variation in the sequence variation data to be analyzed.

The allele frequency data of the variation site in the second variation feature value can be obtained from the included information of various public databases, such as gnomAD (https://gnomad.broadinstitute.org/), EVS (http://evs.gs.washington.edu/EVS), 1000 Genomes Project (http://browser.1000genomes.org), etc. In the present, the source of the allele frequency data of the variation site in the second variation feature value is not limited, including the specific database and the number of databases. The functional prediction data in the second variation feature value can be obtained from the prediction results of various existing functional prediction software, and one or more specific indexes can be selected as the feature value from the prediction results. In addition, if the public database includes the functional prediction data, the corresponding functional prediction data can be directly extracted from the public database. In the present disclosure, the source of the functional prediction data in the second variation feature value is not limited and includes specific software and the number of software, specific indexes, and the number of indexes. The prediction data of the functional prediction software may include, but not limited to, at least one of protein conservation prediction data, nucleic acid conservation prediction data, cleavage hazard prediction data, and variation site harmful degree prediction data. In embodiments of the present disclosure, a second variation feature may be extracted from the public database based on the unique identifier (e.g., cpra) of each variation in the sequence variation data to be analyzed.

S3, the first variation feature set is inputted into a trained first phenotype relationship prediction model, to obtain a first phenotype relationship prediction result, and the second variation feature set is inputted into a trained second phenotype relationship prediction model, to obtain a second phenotype relationship prediction result.

S4, a third phenotype relationship prediction result is obtained by taking a union of the first phenotype relationship prediction result and the second phenotype relationship prediction result.

As a preferred embodiment, after step S4, it may further include: comparing the third phenotype relationship prediction result with a corresponding variation interpretation result; determining, in response to that the third phenotype relationship prediction result is consistent with the variation interpretation result, the variation interpretation result to be reliable; and determining, in response to that the third phenotype relationship prediction result is inconsistent with the variation interpretation result, the variation interpretation result to be unreliable.

The variation interpretation result refers to the phenotype relationship determination result of each variation site of the sequence variation data to be analyzed obtained by different methods based on the third phenotype relationship prediction result. Manual interpretation of results is a common type of variation interpretation result. In addition, by employing customary means in the related art, a variation interpretation result determined to be reliable and a variation interpretation result determined to be unreliable can be added with a self-defined identifier, respectively, to facilitate subsequent data processing. Such a solution can quickly determine the credibility and non-credibility of a large number of variation interpretation results, and handle them separately according to types in the subsequent links. For example, variation sites with a variation interpretation result that is determined to be unreliable can be reviewed, and they are usually determined by manual review. The present solution can greatly reduce the workload of subsequent procedures, thereby saving labor costs and improving the efficiency of interpretation.

Unlike the prior art using a single model for prediction, the technical solution including steps S1 to S4 adopts two relatively independent models (i.e., a first phenotype relationship prediction model and a second phenotype relationship prediction model) for prediction based on different data types, and these two models have complementary effects. The first phenotype relationship prediction model can compensate for false negatives that may be caused by the second phenotype relationship prediction model without functional data of variation sites, co-segregated data, and newly generated data, etc., and the second phenotype relationship prediction model can compensate for false negatives in the first phenotype relationship prediction model caused by phenotype relationship determination results of variation sites that have not been disclosed by variation.

The first phenotype relationship prediction model and the second phenotype relationship prediction model can both be constructed based on a machine learning model, and the construction method is described below.

S31, a data set, including a first data set and a second data set, is constructed. The first data set is a first variation feature of a selected variation site and the reliable phenotype relationship determination result of this variation site, and the second data set is a second variation feature of the selected variation site and the reliable phenotype relationship determination result of this variation site.

The selected variation sites refer to a plurality of variation sites including all types of phenotype relationship determination results. In a preferred embodiment, the reliable phenotype relationship determination result may be the result of an artificial phenotype relationship determination according to industry guidelines or consensus. The reliable phenotype relationship determination result may also be the result that the phenotype relationship determination results of a plurality of disclosed variation sites are consistent.

S32, the first machine learning model is trained and evaluated with the first data set, and the second machine learning model is trained and evaluated with the second data set.

In a preferred embodiment, the first machine learning model and the second machine learning model may be selected from at least one of logistic regression, naive Bayes, support vector machines, and artificial neural networks.

S33, a first phenotype relationship prediction model is determined based on the evaluation result of the first machine learning model, and a second phenotype relationship prediction model is determined based on the evaluation result of the second machine learning model.

In a preferred embodiment, the first phenotype relationship prediction model is a logistic regression model, and the second phenotype relationship prediction model is a neural network model.

When the present solution is applied to the analysis of human Mendelian genetic disease-related variation, the specific process is as follows.

In this embodiment, the first phenotype relationship prediction model is used to quickly perform pathogenicity determination on the recorded variations in a public database with on a uniform standard. The construction process of the first phenotype relationship prediction model includes the following step.

A2, a first data set is constructed, the first data including the results of the pathogenicity determination recorded in the public variation database and the results of the pathogenicity determination made by the interpretation experts.

The public variation database may include at least one of ClinVar, HGMD, LOVD, and UMD (http://umd-predictor.eu/).

Specifically, after the variation data are collected from the common variation database, the variation data collected from the respective common variation databases can be annotated by using a variation annotation tool, to obtain cpra information thereof, and the cpra information serves as the unique identifier of the variation. The variation data of the respective public variation databases are summarized with the index of cpra of variation.

At the same time, according to the American College of Medical Genetics and Genomics (ACMG) Variation Interpretation Guidelines, the interpretation experts perform pathogenicity determination on the same variation in combination with a population frequency database, prediction software, medical literature, and other information, and the determination result can be regarded as a reliable phenotype relationship determination result, which is used as the standard result of training data.

Furthermore, it is required to establish the unique mapping relationship of the pathogenicity determination results recorded in the public variation database, the pathogenicity determination results made by interpretation experts, and the characters satisfying the input requirements of the machine learning model.

The structure and contents of the first data set are shown in Table 1.

[Table 1]

| chr-pos-ref-alt | ClinVar pathogenicity determination | HGMD pathogenicity determination | LOVD pathogenicity determination | UMD pathogenicity determination | pathogenicity determination by interpretation experts |
|---|---|---|---|---|---|
| chr8-90976737-T-C | Likely_pathogenic | DM | pathogenic | 5-Causal | pathogenic |

Since the pathogenicity determinations of the respective common variation databases and interpretation experts are ordered discrete data, the present disclosure converts the character data in the first data set into numeric data, as shown in Table 2.

[Table 2]

| chr-pos-ref-alt | ClinVar pathogenicity determination | HGMD pathogenicity determination | LOVD pathogenicity determination | UMD pathogenicity determination | pathogenicity determination by interpretation experts |
|---|---|---|---|---|---|
| 8-90976737-T-C | 4 | 5 | 5 | 5 | 5 |

B2, the data in the first data set are randomly divided into a first training set and a first test set according to a first preset ratio.

The first preset ratio may be 8: 2.

C2, the first machine learning model is trained using the data in the first training set, and the trained first machine learning model is evaluated using the first test set.

The first machine learning model is selected from at least one of logistic regression, a Naive Bayes, a support vector machine, and an artificial neural networks machine learning model.

D2, a first phenotype relationship prediction model is determined from a plurality of trained first machine learning models based on the evaluation results.

Specifically, the first phenotype relationship prediction model aims to output the unique variation pathogenicity determination based on the variation pathogenicity determinations of multiple different common variation databases. The variation pathogenicity determination is a multi-classification problem with fewer features, such that machine learning models such as logistic regression, naive Bayesian, support vector machines and artificial neural networks can be selected to realize the above function.

As an embodiment, the multiple different machine learning models may be selected, the respective selected machine learning models may be independently trained based on a first training set, and the performance of the respective trained machine learning models may be evaluated based on a first test set. The evaluation indicators may include accuracy rate, precision rate, recall rate, etc. Based on the performance evaluation results of the respective models, the model with the best performance is selected as the first phenotype relationship prediction model used in the present disclosure. For example, the trained model with the highest accuracy rate can be used as the first phenotype relationship prediction model.

When the first phenotype relationship prediction model is used to predict the phenotype relationship, the first feature value of each variation can be extracted. The first phenotype relationship prediction result can be obtained by inputting the first variation feature value into a first phenotype relationship prediction model.

It should be noted that the first phenotype relationship prediction model outputs a prediction result for the variation included in the public database based on a preset phenotype relationship. As an example, for the human Mendelian genetic disease-related variation pathogenicity determination, the preset phenotype relationship usually has three different types, i.e., pathogenic or possibly pathogenic, benign or possibly benign, with unclear meaning; or five different types, i.e., pathogenic, possibly pathogenic, benign, possibly benign, with unclear meaning, which is not specifically limited in the present disclosure.

Accordingly, the construction process of the second phenotype relationship prediction model may include: A3, constructing a second data set. The second data set includes allele frequency data of the variation site, functional prediction data, and reliable phenotype relationship determination results.

Specifically, allele frequency data can be derived from gnomAD, the Millennium Database, the ExAC Database, etc.; Functional prediction data can be derived from the data of various prediction software, such as SIFT, Polyphen2, MutationTaster, GERP++, DANN, etc. It can be seen therefrom that, the second phenotype relationship prediction model differs from the first phenotype relationship prediction model in that the second phenotype relationship prediction model is obtained based on the original data for judging the phenotype relationship, while the first phenotype relationship prediction model is obtained based on the disclosed phenotype relationship determination result.

The variation sites in the second data set are required to have consistent phenotype relationship determination results at a plurality of disclosed variation sites. The consistent determination results are reliable phenotype relationship determination results, and the results are used as standard results of training data.

Further, it is required to establish a unique mapping of allele frequency data, functional prediction data, and the reliable phenotype relationship determination results to characters satisfying the input requirements of the machine learning model.

B3, the data in the second data set are randomly divided into a second training set and a second test set based on a second preset ratio.

The second preset ratio may be 8:2.

C3, the second machine learning model is trained using the data in the second training set, and the trained second machine learning model is evaluated using the second test set.

As the second machine learning model, machine learning models such as logistic regression, naive Bayesian, support vector machine, and artificial neural networks can be selected.

D3, an optimal second machine learning model is determined and trained based on the evaluation result, and the optimal second machine learning model is used as a second phenotype relationship prediction model.

When the phenotype relationship is predicted with the second phenotype relationship prediction model, the second variation feature value of each variation can be extracted; and a second phenotype relationship prediction result can be obtained by inputting the second variation feature value into the second phenotype relationship prediction model.

In an embodiment of the present disclosure, in practice, variation interpretation results can be obtained by means of manual interpretation based on the sequence variation data to be analyzed. At the same time, a first phenotype relationship prediction result is obtained by inputting corresponding data of the same variation site into a trained first phenotype relationship prediction model; a second phenotype relationship prediction result is obtained by inputting the trained second phenotype relationship prediction model; and a union of the first phenotype relationship prediction result and the second phenotype relationship prediction result is obtained as a third phenotype relationship prediction result. The variation interpretation result can be compared with the corresponding the third phenotype relationship prediction result. If these two results are consistent, the variation interpretation result is determined to be reliable. Otherwise, the variation interpretation result is determined to be unreliable. A manual review is required for the unreliable variation interpretation results.

It should be noted that the reliable phenotype relationship determination results above for training are considered to be accurate and small; the interpretation results for comparison with the third phenotype relationship prediction result can be a phenotype relationship determination result for each variation site of the sequence variation data to be analyzed, which is obtained by any different method to the third phenotype relationship prediction result, such as a manual interpretation result of a person skilled in the art.

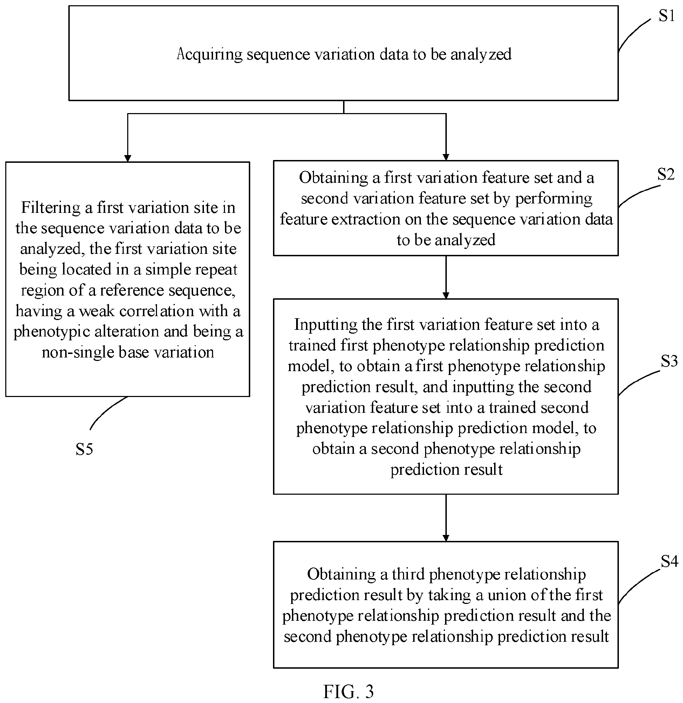

In an embodiment of the present disclosure, as illustrated in FIG. 3, the sequence variation analysis method may further include the following step.

S5, a first variation site in the sequence variation data to be analyzed is filtered. The first variation site is located in a simple repeat region of a reference sequence, has a weak correlation with a phenotypic alteration, and is a non-single base variation.

The simple repeat region refers to a repeating region composed of 1-5 base repeating units, such as AAA, CAACAACAACAA. Weak correlation with the phenotypic alteration means that the variation is located in a non-coding region or intron region and that the allele frequency of the variation is greater than 0.05. Variation located in a non-coding region or an intron region may refer to functional annotation of the variation as "-" (meaning no functional annotation), "intron", "3'-UTR" (meaning untranslated region at the 3' end), "5'-UTR" (meaning untranslated region at the 5' end), "no change" (no change compared with the reference mRNA sequence).

As an example, the first variation site further satisfies any of the following conditions: Condition 1: the total number of detections of the first variation site being greater than a first preset value, and the number of low-quality detections being greater than a second preset value; and Condition 2: locating within a third preset value of upstream and downstream of the physical position of a reference sequence of the variation site satisfying Condition 1.

As an example, a first variation site data set including a first variation site may be pre-established and constructed as follows.

A1, the sequence variation data to be analyzed of multiple samples are acquired.

Specifically, the step is the same as the aforementioned step S1. More specifically, sample sequencing data on a fixed sequencing chip (e.g., LCY171 chip) on a gene sequencer (e.g., MGISEQ-2000 sequencer) can be collected, and the variation site and corresponding unique variation identifier from the sample sequencing data.

It should be noted that in actual use, the data set of the first variation site is continuously updated with the accumulation of samples, so as to realize the filtration of the sequence variation data to be analyzed.

B1, It is judged whether each obtained variation site is located in a simple repeat region of a reference sequence, and if so, a unique variation identifier corresponding to the variation site is recorded.

C1, the results of B1 are filtered to obtain a variation site satisfying a preset filtering condition.

The variation sites satisfying the preset filtering conditions can be: variations with a functional annotation of "-" (indicating no functional annotation), "intron", "3'-UTR" (indicating untranslated region at 3' end), "5'-UTR" (indicating untranslated region at 5' end), or "no change", and having frequency greater than 0.05 (indicating probability of variation in the population).

Specifically, filtering can be performed based on the variation function and variation frequency, and the filtering conditions are the functional annotation of "-", "intron", "3'-UTR", "5'-UTR", or "no change", and having the frequency greater than 0.05. The filtering conditions are set in order to select variations that have a high probability of not causing a phenotypic alteration of the gene, i.e., high-frequency variations located in non-exon regions.

D1, the number of low-quality detections and the total number of detections of each variation site in the results obtained in step C1 are statistically calculated.

Specifically, after the processing in step C1, the number of low quality detections, the number of high-quality detections, and the total number of detections using the unique variation identifier determination as an index are statistically calculated. The total number of detections is the sum of the number of low-quality detections and the number of high-quality detections.

E1, the data set of the first variation site is constructed by using the variations satisfying: the total number of detections greater than the first preset value, the number of low-quality detections greater than the second preset value, and as a non-single base variation.

Specifically, the statistical rules are: 1) the total number of detections ≥ a first preset value (e.g., 8), the number of low-quality detections ≥ a second preset value (e.g., 1), and the variation site of non-single base variation; and 2) searching the variation sites of non-single base variation within a third preset value (e.g., 3 bp) upstream and downstream of the physical position of the reference sequence of the variation site in 1) and still located in the simple repeat region. It should be noted that in the simple repeat region, the position of the alignment between the variation site and the reference sequence may fluctuate, and different databases or analysis software may record in different ways, but they are substantially the same variation. For example, the reference sequence ATATAT, the variation site sequence AT, and the variation site sequence may be matched to the first, third, and fifth positions of the reference sequence, respectively. Statistical rule 2) is therefore set to ensure a comprehensive including of the first variation site. The first variation site data set is composed of variation sites satisfying any of the above statistical rules 1) and 2).

Further, when filtering a first variation site in the sequence variation data to be analyzed, a unique variation identifier of each variation site in the sequence variation data to be analyzed can be obtained first, and filtering can be performed based on the unique variation identifier. The specific analysis process can be: invoking the first variation site data set, and traversing the first variation site data set based on the unique variation identifier of the sequence variation data to be analyzed. If there are consistent unique variation identities, it indicates that the variation sites in the sequence variation data to be analyzed are the first variation sites required to be filtered; otherwise, it is reserved.

As an example, the data for phenotype relationship prediction may be the data after the filtering operation of step S5, i.e., steps S2 to S4 may be performed after step S5 using the filtered data of step S5. Thus, unnecessary prediction can be avoided by reducing the data of phenotype relationship prediction, thereby improving the prediction efficiency.

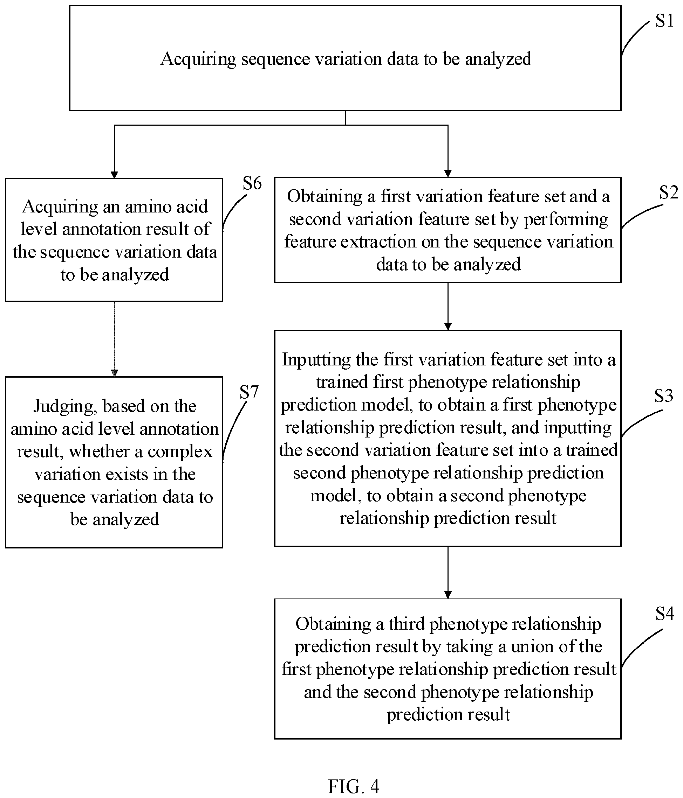

In an embodiment of the present disclosure, as illustrated in FIG. 4, the sequence variation analysis method, the method may further include the following steps.

S6, an amino acid level annotation result of the sequence variation data to be analyzed is acquired.

S7, it is judged based on the amino acid level annotation result whether a complex variation exists in the sequence variation data to be analyzed.

The complex variations may include insertion and replacement composite events, deletion, and deletion composite events, composite events, deletion and replacement composite events, and various complex variations have no limitation on the number of each specific variation. In addition, custom identifiers may be added to sites that the complex variation is present and/or absent, for subsequent data processing by means customary in the art.

As an example, the variation sites where the complex variation exists satisfy the following condition: at least two of variation site reference sequence coordinates resulting in an amino acid change being overlapped; or at least two of the variation site reference sequence coordinates resulting in the amino acid change being adjacent and affecting the same encoded amino acid.

When a variation is detected using a variation detection tool (e.g., GATK software), only a single replacement event or insertion event will be detected, and composite events including insertion and replacement cannot be detected. In order to remedy this defect, in the present embodiment, the variation annotations detected by the variation detection tool are subjected to the judgment of complex variation, and specifically, judging whether the variations at adjacent positions of the coding region of the same gene affect the coding of the same amino acid residue. If there are complex variations, the review is required, for example, in combination with manual interpretation, and *vice versa.*

Specifically, the amino acid level annotation result is acquired from the sequence variation data to be analyzed, and the annotation result is usually named using HGVS naming rules. For the variation with amino acid change, it is determined that there is a complex variation when any of the following conditions is satisfied: 1) reference sequence coordinates of at least two variations are overlapped; and 2) reference sequence coordinates of at least two variations are adjacent and affect the same amino acid coding. In condition 1), "coordinates" refer to the position of the reference sequence corresponding to the variation, and "overlapped" refers to the overlap of the reference sequence coordinates greater than or equal to 1 bp. Such a condition allows for as comprehensive a screening of complex variations as possible. In condition 2), "adjacent" means within 4 bp upstream and downstream of the reference sequence. Such a condition can accurately screen for complex variations. Therefore, it can remedy the defects of the existing variation detection tools by judging the complex variation and combining the complex variations, thereby improving the efficiency of variation interpretation.

As an example, the sequence variation data to be analyzed in step S6 may be the sequence variation data to be analyzed in step S1 or the sequence variation data to be analyzed with a determination result after the third phenotype relationship prediction result is compared with the corresponding variation interpretation result.

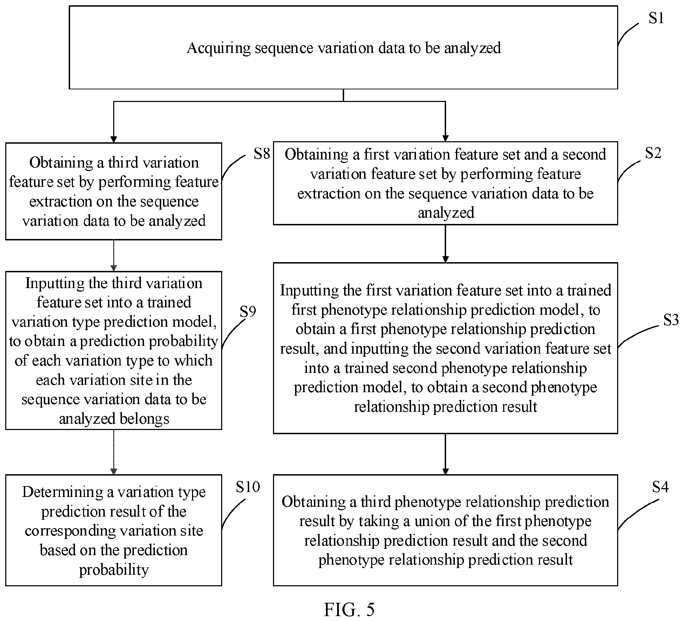

In an embodiment of the present disclosure, as illustrated in FIG. 5, the sequence variation analysis method may further include the following step.

S8, a third variation feature set is obtained by performing feature extraction on the sequence variation data to be analyzed.

The third variation feature value in the third variation feature set may include variation support data (when the sequence variation data to be analyzed is obtained by sequencing means, the variation support data may include quality value, sequencing depth, reads ratio supporting variation), and allele frequency (such as ESP6500_MAF, G1000_AF).

S9, the third variation feature set is input into a trained variation type prediction model, to obtain a prediction probability of each variation type to which each variation site belongs in the sequence variation data to be analyzed.

The variation types may include homozygous variation, heterozygous variation, and no variation. No variation can be homozygous genotype for the reference sequence at this site.

S10, a variation type prediction result of the corresponding variation site is determined based on the prediction probability .

Specifically, the variation type prediction result can be determined by: comparing the prediction probability of respective variation types; determining the variation type with the maximum prediction probability; comparing the maximum prediction probability with a preset threshold; and determining, if the prediction probability is greater than a preset threshold, that the variation type corresponding to the maximum prediction probability is the variation type prediction result of the variation site.

As an example, the construction process of the variation type prediction model may include the following steps.

A4, a third data set is constructed. The third data set includes quality values, sequencing depths, reads ratio supporting variation, allele frequency, and reliable variation type determination results.

The reliable variation type determination result may be a result obtained by using an intra-industry consensus gold standard verification means, for example, a Sanger verification result.

B4, the data in the third data set are randomly divided into a third training set and a third test set based on a third preset ratio.

The third preset ratio may be 8:2.

C4, the third machine learning model is trained using the data in the third training set, and the trained third machine learning model is evaluated using the third test set.

Specifically, the variation type prediction model is constructed with prior probability and conditional probability required to compute a Bayesian classifier.

D4, a third phenotype relationship prediction model is determined from the trained plurality of third machine learning models based on the evaluation result.

A model test is performed on the variation type prediction model based on the data in the third test set, the classification of the test set data is determined based on the variation type prediction model, and the accuracy of the variation type prediction model is obtained based on the determined correct quantity.

Specifically, the variation sites verified by Sanger are selected as training samples and relevant data thereof are collected to compose a third training set. The training samples are divided into a total of $K$ variation types $c_1, c_2, ... , c_K$. The obtained variation data are preprocessed to acquire relevant feature values of the variation information, which are recorded as $x_i$. The prior probability of each feature value is calculated based on the selected training set, and the prior probability can be estimated based on the number of occurrences of various types of samples in the third training set. Taking the feature value x1 with the variation type c1 as an example, the prior probability thereof is:

$$P(x_i = x_1 | c_k = c_1) = \frac{\text{the total amount of data for } x_i = x_1}{\text{the total amount of data}}.$$

For each class $c_k$, the prior probability $P(c_k)$ and class conditional probability are computed based on the third training set, and the conditional probability $P(c_k|x)$ for a given training feature x is estimated using a Bayesian formula, i.e.,

$$P(c_k|x) = \frac{P(c_k) \ \prod_{i=1}^{n} P(x_i|c_k)}{P(x)},$$

where $P(x)$ is the evidence factor used for normalization, and the value of all classes is a constant.

The performance of the above machine learning model is evaluated using the third test set, and the evaluation indexes may include accuracy rate, precision rate, and recall rate.

During the use of this model, the constructed variation type prediction model of the variation to be tested is used for prediction, to obtain the probability $P(c_k|x)$ of each class. Taking $P_{max}(c_k|x)$, it is determined that the data $x$ belongs to the corresponding class $c_k$. Alternatively, a quality control threshold is set, and only if $P_{max}(c_k|x)$ is greater than the preset threshold, the data $x$ is determined to belong to the corresponding class $c_k$.

As an example, the sequence variation data to be analyzed in step S8 may be the sequence variation data to be analyzed in step S1, or the sequence variation data to be analyzed with a determination result after the third phenotype relationship prediction result is compared with the corresponding variation interpretation result, or the sequence variation data to be analyzed with a complex variation result indicating whether a complex variation exists or not.

As a preferred embodiment, subsequent to step S10, the method further includes: determining, when the variation type prediction result only includes a reference sequence genotype, the variation site to be unreliable; and determining, when the variation type prediction result includes at least one non-reference sequence genotype, the variation site to be reliable. Furthermore, custom identifiers may be added to the variation sites that are determined to be unreliable and/or reliable by customary means in the art, for subsequent data processing. The present solution can quickly determine the credibility and non-credibility of a large number of variation sites, and process them separately based on types in the subsequent procedures. In an example, only variation sites determined to be unreliable are verified. In another example, only variation sites associated with phenotypic alterations and determined to be unreliable are verified, where the variation sites associated with phenotypic alterations may be pathogenic variations. The verification is typically performed in a gold-standard manner recognized in the art, e.g., first-generation sequencing verification. The present solution can greatly reduce the workload of subsequent verification, reduce the labor cost, and improve the interpretation efficiency.

Thus, the sequence variation analysis method of the present disclosure can quickly determine whether the variation needs to be reviewed or experimentally verified, etc. To a certain extent, the cost of variation experiment verification is reduced for the whole interpretation process, and the results of manual interpretation or automated interpretation are analyzed to rapidly output the variation required to be reviewed, thereby reducing the cost of manual review and ensuring the accuracy of results.

The sequence variation analysis method according to the embodiments of the present disclosure is described below in conjunction with a specific example.

In the specific example, taking human Mendelian genetic disease variation analysis as an example, 7937 variation interpretation data from 599 samples from clinical data were selected as sequence variation data to be analyzed, and the clinical data should be acquired with legal compliance. The specific implementations are described below.

1 First variation site filtering

1.1 Construction of first variation site data set

1.1.1 Sample sequencing data of a total of 3038 samples on the LCY171 sequencing chip on the MGISEQ-2000 sequencer were collected continuously and variations located in the simple repeat region were labeled as "yes". For example, chr1-241663902-T-TGAGAGAGA, the reference sequence has "GA" repeats, labeled as "yes".

1.1.2 The variation sites labeled as "yes" were filtered based on variation function and allele frequency. The filtering conditions set in the embodiment were: (1) variation with functional annotation as "-", "intron", "3-UTR", "5-UTR", "or no change"; and (2) variation having a frequency greater than 0.05, which can be determined through G1000, ESP6500, genomAD and other population databases.

For example, the allele frequency of the variation recorded in the three databases of G1000, ESP6500, and genomAD for the variation chr11-108150207-CT-C was greater than 0.05, so the variation was filtered.

1.1.3 The number of low-quality detections, the number of high-quality detections, and the total number of detections were counted for the variation sites retained after filtering in 1.1.2. The total number of detections is the sum of the number of low-quality detections and the number of high-quality detections.

1.1.4 Construction of first variation site data set

The first variation site data set was constructed by including variation sites satisfying one of the following two conditions. At least cpra information of the included variation sites shall be recorded in the first variation site data set.

Condition (1): labeled as "yes", with the total number of detections $\geq 8$, the number of low-quality detections $\geq 1$, and non-single base variation (i.e., the number of ref bases $\neq$ the number of alt bases); and

Condition (2): the number of ref bases $\neq$ alt bases and labeled as "yes", searched within 3 bp nearby in those meeting condition (1).

1.2 Searching and filtering the first variation site

Variation cpra information of the data to be analyzed was extracted, and the first variation site data set was traversed. If the cpra was consistent with a cpra in the data set, "Yes" was output, indicating that there was a first variation site, i.e., the variation to be filtered; otherwise, "No" was output, indicating that there was no a first variation site, i.e., the variation to be retained.

2 Determination of complex variation

The specific implementations are described below.

2.1 It was judged based on result indicating the presence or absence of pHGVS point whether there was an amino acid change in the variation.

In some embodiments, the determination of complex variation can also process the received data (e.g., input data in VCF format, FASTQ format, etc.) using bioinformatics software (e.g., GATK, ANNOVAR, alamut, etc.) to perform amino acid level annotation to determine whether a variation contained a result of a pHGVS point. For example, for variation chr7-142458526-A-G, the amino acid level annotation result was p.Asn54Ser, i.e., containing a pHGVS point.

2.2 The position of the variation with amino acid change was determined to judge whether there was the same gene variation with two or more adjacent coding regions. For example, the detected variation includes both chr7-142458526-A-G (c.161A>G, p.Asn54Ser) and chr7-142458527-C-G (c. 162C>G, p.Asn54Lys), the former is cDNA 161 and the latter is 162, which are adjacent to each other.

2.3 Based on the variation type of adjacent variation and the position of amino acid change, it was determined whether there was complex variation. The above two variations (i.e., chr7-142458526-A-G, chr7-142458527-C-G) are both Single Nucleotide Polymorphism (SNP). Based on pHGVS, the position of amino acid change led to the change of amino acid Asn at position 54, and these two variations both have the same amino acid change position, which was determined to be a complex variation.

The variation sites determined to have complex variations were identified with custom characters. A combined interpretation can be performed subsequently to improve the efficiency of interpretation.

3 Phenotype relationship prediction

3.1 First phenotype relationship prediction model

3.1.1 Construction

3.1.1.1 Training data collection

A total of over 30,000 variations and their determination of pathogenicity were acquired from the public variation databases ClinVar and HGMD as training data.

3.1.1.2 Training data preprocessing and feature extraction

(1) Training data preprocessing

The training data were annotated using the variation annotation tool to obtain the cpra information thereof (genome version was hg19), and the information was used as the unique variation identifier. Using the cpra of variation an index, the variation and its pathogenicity determination of the public variation database were summarized into a training summary database. According to the ACMG variation interpretation guidelines, combined with the population frequency database, prediction software, and medical literature, the interpretation experts artificially determined the pathogenicity of the variations in the summary database. The determination results were used as the standard results of training data. The preprocessed training summary database structure and contents were shown in Table 3.

[Table 3]

| Chromosome number -genome coordinates-reference sequence-alter sequence | ClinVar pathogenicity determination | HGMD pathogenicity determination | Pathogenicity determination by experts |
|---|---|---|---|
| 13-32968886-G-A | Pathogenic | DM | Pathogenic/ Likely Pathogenic |

(continued)

| Chromosome number -genome coordinates-reference sequence-alter sequence | ClinVar pathogenicity determination | HGMD pathogenicity determination | Pathogenicity determination by experts |
|---|---|---|---|
| 13-32893467-G-A | Pathogenic/Likely_pathogenic | DM | Pathogenic/ Likely Pathogenic |
| 13-32971167-G-C | Benign/Likely_benign | DM? | VLTS |
| 14-75485594-C-T | Uncertain_significance | DM? | VUS |
| 7-6031639-T-C | Conflicting_interpretations_ of_ pathogenicity (submission without pathogenic) | DP | VLTS |
| 2-48033981-T-TTTGA | Likely_benign | FP | VUS |
| 16-89849480-C-T | Benign | DP | Benign/ Likely_ benign |
| 2-47643457-G-A | Benign | FP | Benign/ Likely_ benign |
| ... | ... | ... | ... |

(2) Training data feature extraction

Since the pathogenicity determination results of the public variation database and the interpretation experts were all ordered discrete data, the character data in the summary database could be converted into numerical data based on the corresponding relationships shown in Tables 4 to Table 6 to complete the construction of the first training set. The structure and content of the first training set after feature extraction was shown in Table 8.

[Table 4]

| HGMD pathogenicity determination (character) | HGMD pathogenicity determination (numeric) |
|---|---|
| Null (no such variation recorded in the database) | 0 |
| R | 1 |
| FP | 2 |
| DP | 3 |
| DFP | 4 |
| DM? | 5 |
| DM | 6 |

[Table 5]

| ClinVar pathogenicity determination (character) | ClinVar pathogenicity determination (numeric) |
|---|---|
| Null (no such variation recorded in the database) | 0 |
| not_provided | 1 |
| risk_factor | 1 |
| drug_response | 1 |
| other | 1 |
| Benign | 2 |

(continued)

| ClinVar pathogenicity determination (character) | ClinVar pathogenicity determination (numeric) |
|---|---|
| Benign/Likely_benign | 3 |
| Likely_benign | 4 |
| Conflicting_interpretations_of_pathogenicity (submission without pathogenic) | 5 |
| Uncertain_significance | 6 |
| Conflicting_interpretations_of_pathogenicity (submission with pathogenic) | 7 |
| Likely_pathogenic | 8 |
| Likely_pathogenic,_risk_factor | 8 |
| Pathogenic/Likely_pathogenic | 9 |
| Pathogenic/Likely_pathogenic,_risk_factor | 9 |
| Pathogenic | 10 |

[Table 6]

| Pathogenicity determination by interpretation experts (character) | Pathogenicity determination by interpretation experts (numeric) |
|---|---|
| Pathogenic/Likely Pathogenic | 3 |
| VUS | 2 |
| Benign/Likely Benign | 1 |

[Table 7]

| Chromosome number -genome coordinates-reference sequence-alter sequence | ClinVar pathogenicity determination | HGMD pathogenicity determination | Pathogenicity determination by experts |
|---|---|---|---|
| 13-32968886-G-A | 10 | 6 | 3 |
| 13-32893467-G-A | 9 | 6 | 3 |
| 13-32971167-G-C | 3 | 5 | 2 |
| 14-75485594-C-T | 6 | 5 | 2 |
| 7-6031639-T-C | 5 | 3 | 2 |
| 2-48033981-T-TTTGA | 4 | 2 | 2 |
| 16-89849480-C-T | 2 | 3 | 1 |
| 2-47643457-G-A | 2 | 2 | 1 |
| ... | ... | ... | ... |

3.1.1.3 Model selection and training

The first training set was randomly divided into a first training set and a first test set at a first preset ratio, such as 8:2. The first training set was used for training through a logistic regression model.

3.1.1.4 Model evaluation and determination

The logistic regression model was trained by the first training set and evaluated by the first test set with an accuracy rate of 91.0%, and the test performance data were shown in Table 8.

[Table 8]

| Performance index | Precision rate | Recall rate | f1 score |
|---|---|---|---|
| 1 | 0.91 | 0.84 | 0.87 |
| 2 | 0.87 | 0.92 | 0.90 |
| 3 | 0.97 | 0.97 | 0.97 |
| | | | |
| Macro average | 0.92 | 0.91 | 0.91 |
| Weighted average | 0.91 | 0.91 | 0.91 |

3.1.2 Application

3.1.2.1 Prediction data collection

Variations to be predicted, e.g., NM_000267.3(NF1):c.1722-2A>G, NM_000057.4(BLM):c.893C>T (p.Thr298Met) and NM_000244.3(MEN1):c.670-6C>T, and the pathogenicity determination thereof in the public variation databases ClinVar and HGMD were collected as data to be predicted. Alternatively, the variation to be predicted may be a variation site taken after filtering the first variation site as described above.

It should be noted that if the variation to be predicted had no results in either of these two common variation databases, then the variation cannot be used for pathogenicity determination through the present disclosure.

3.1.2.2 Training data preprocessing and feature extraction

The variation to be predicted was annotated with the variation annotation tool to obtain the cpra information thereof (genome version was hg19), and the information was used as the unique identify of variation. Pathogenicity determinations of variation to be predicted in public variation databases ClinVar and HGMD were summarized in one database by using cpra of variations as the index. Since the pathogenicity determination results in the public variation database were ordered discrete data, the character data in the prediction summary database were converted into numerical data based on the conversion logic shown in Table 8, and the prediction data processing was completed. The structure and content of the preprocessed and feature-extracted prediction summary database were shown in Table 9.

[Table 9]

| Chromosome number -genome coordinates- reference sequence-alter sequence | ClinVar pathogenicity determination | HGMD pathogenicity determination |
|---|---|---|
| 17-29550460-A-G | 6 | 10 |
| 15-91295110-C-T | 5 | 3 |
| 11-64575158-G-A | 5 | 5 |

3.1.2.3 Model result output

The data in the prediction summary database were converted into an input format accepted by the model, and the corresponding data were input into the trained first phenotype relationship prediction model, to obtain the variation pathogenicity determination of the above-mentioned three variations to be predicted, namely, the first phenotype relationship prediction result, and the results were shown in Table 10. The model can be obtained by conversion of numeric and character data as shown in Tables 4 and Table 5. NM_000267.3(NF1):c.1722-2A>G, NM_000057.4(BLM):c.893C>T (p.Thr298Met) and NM_000244.3(MEN1):c.670-6C>T were predicted as Pathogenic/Likely Pathogenic, Benign/Likely benign, and VUS, respectively.

[Table 10]

| Chromosome number -genome coordinates-reference sequence-alter sequence | ClinVar pathogenicity determination | HGMD pathogenicity determination | Model prediction pathogenicity determination |
|---|---|---|---|
| 17-29550460-A-G | 6 | 10 | 3 |
| 15-91295110-C-T | 5 | 3 | 1 |
| 11-64575158-G-A | 5 | 5 | 2 |

3.2 Second phenotype relationship prediction model

3.2.1 Construction

3.2.1.1 Variation data with clear pathogenicity results interpreted by experts were acquired.

Specifically, variation data could be taken from the ClinVar database, the ClinVar database (VCF_GRCH37, fileDate = 2017-10-29) and contained nearly 300,000 human genetic variations, covering various classes of variation from pathogenic to benign. The ClinVar database contained 46,585 defined disease sites. A total of 21,105 variation sites regarded as "DM" in the HGMD database and in dbNSFP (https://sites.google.com/site/jpopgen/dbNSFP) was screened, half of which, i.e., 10,552, were selected as pathogenic variations in the second training set, and the other of which, i.e., 10,553, pathogenic sites constituted the first test subset of the second test set. The ClinVar database contained 23,892 benign sites, and a total of 4,664 sites in the dbNSFP database were selected as benign variations in the second training set. To deal with the missing value in the dbNSFP database, the value was directly assigned to 0.

3.2.1.2 The variation data in the second training set and the first test subset were preprocessed, and the cpra of the variation was taken as a unique identifier and index of the variation, to acquire a variation information feature value $x_n$ used for training. In a specific example, the model was based on allele frequency databases 1000 Genomes Project, ESP6500, and multiple functional prediction software data. Specifically, functional prediction software can be classified as for protein conservation prediction such as SIFT, Polyphen2, MutationTaster, for nucleic acid conservation prediction such as GERP ++, for splice hazard prediction such as dbscSNV, and for variation site hazard prediction such as DANN. One or more technical indicators of each function prediction software were extracted as feature values to acquire a plurality of feature values of variation (counted as $x_n$). The specific feature values are SIFT_converted_rankscore, Polyphen2_HDIV_rankscore, Polyphen2_HVAR_rankscore, LRT_converted_rankscore, LRT_Omega, MutationTaster_converted_rankscore, MutationAssessor_score_rankscore, FATHMM_converted_rankscore, PROVEAN_converted_rankscore, MetaSVM_rankscore, MetaLR_rankscore, REVEL_score, CADD_raw_rankscore, DANN_rankscore, GERP++_RS_rankscore, splicing_consensus_ada_score, splicing_consensus_rf_score, phyloP100way_vertebrate_rankscore,phyloP20way_mammalian_rankscore, phastCons100way_vertebrate _rankscore,phastCons20way _mammalian_rankscore, SiPhy_29way_logOdds _rankscore,1000Gp3_AF, ESP6500_AA _AF, respectively. Details of optional specific features can refer to: https://drive.google.com/file/d/1Vse3b_qw_E46eDcsuLegF5HxpodAZ6Og/view, and https://drive.google.com/file/d/12-sSAB_hP9fWNxvJZ6IpMZvGXCVYmfns/view.

3.2.1.3 Construction of second machine learning model. Based on the feature values $x_n$ related to the variation information and the unique pathogenicity determination of the variation, machine learning models such as logistic regression, Naive Bayes, support vector machine, and artificial neural networks were selected as the second machine learning model to realize the prediction function.

In a specific example of the present disclosure, a neural network model may be selected as the second machine learning model. The constructed neural network model can contain three hidden layers, the number of nodes was 16, 128, and 16, respectively; the weight initialization function is uniform, the activation function is hard_sigmoid, the optimizer adopts the default parameters of the Adadelta method, the neuron inactivation probability dropout_rate is 0.05, the number of training iterations is 800, and the number of samples selected for one-time training batch_size is 64.

3.2. a4 Model results evaluation

The second machine learning model obtained by training can be evaluated by two independent test subsets to test and verify the accuracy of the model. Test subset 1 was the above-mentioned first test subset, and test subset 2 was a variation result interpreted by ACMG based on the accumulated clinical interpretation. The sites that were clinically definitely determined as Pathogenic (pathogenic variation) and Benign (benign variation) were screened. The result with the most interpretation times was selected for the site that were repeatedly interpreted with inconsistent interpretation results. After screening the variations in the dbNSFP database and removing the variations in the training set, a total of 728 variations were obtained, including 618 pathogenic variations and 108 benign variations.

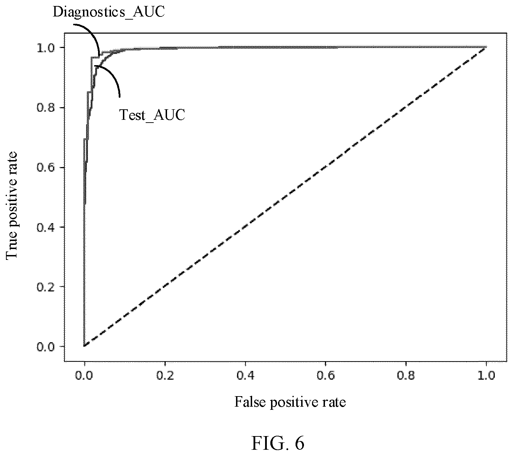

The performance of the trained second machine learning model was evaluated by the above two test subsets. There were 10,553 pathogenic sites in the ClinVar database with an accuracy of 98.77%, and 728 clinical sites with an accuracy of 97.79%. The Receiver Operating Characteristic (ROC) results were illustrated in FIG. 6, with a predicted AUC of 0.99 for 728 clinical sites. In a similar method, the best effect of REVEL test was AUC = 0.957; the best effect of MLP test was AUC = 0.94; the best effect of the DANN test was AUC = 0.9459. Obviously, the prediction submodel of the present disclosure can improve by 4 to 5 percentage points compared with the AUC of the known method, and thus it can assist genetic analysis for rapid pathogenicity review for quality control of variation pathogenicity, with higher accuracy.

3.2 b Application

3.2. b1 The feature values of the variation to be predicted, i.e., the feature values $x_n$ used by the variation to be predicted in the training data, were collected, in particular the same feature values as described in 3.2.1.2.

The variation to be predicted may be a variation site taken after filtering the first variation site as described above.

3.2. b2 the feature values of the variation were input into a second phenotype relationship prediction model, to obtain a phenotype relationship prediction result of the variation to be predicted, i.e., a second phenotype relationship prediction result.

3.3 A third phenotype relationship prediction result was obtained by taking the union of the first phenotype relationship prediction result and the second phenotype relationship prediction result. Comparing the third phenotype relationship prediction result with a corresponding variation interpretation result, when the third phenotype relationship prediction result was consistent with the variation interpretation result, the variation interpretation result was determined to be reliable; and when the third phenotype relationship prediction result was inconsistent with the variation interpretation result, the variation interpretation result was determined to be unreliable. The variation with the unreliable variation interpretation result needed to be further manually reviewed.

4 Variation type prediction

4.1 Model construction

4.1.1 A training set was built. A total of 843 variation sites were selected from the historical sequencing data of sanger-verified variation results and randomly divided into a third training set and a third test set based on the ratio of 4:1. The third training set was used to train the model and the third test set was used to test the model. The specific data distribution for the training set with 674 variation sites was: 269 non-variation sites, 212 homozygous variation sites, and 193 heterozygous variation sites.

4.1.2 The obtained 674 variation site data were preprocessed to acquire relevant feature values $x_n$ of the variation information. The selected feature values may include the five-dimensional characteristics of $x_1$ quality value, $x_2$ sequencing depth, $x_3$ reads ratio supporting variation, $x_4$ ESP6500_MAF (allele frequency) and $x_5$ G1000_AF (allele frequency). For example, the variation chr2-223086092-G-A was verified as heterozygous variation by Sanger, and the feature values thereof were preprocessed as: $x_1 = 99$; $x_2 = 250$; $x_3 = 0.4480$; $x_4 = 0.000077$; $x_5 = 0.001597$; 4.1.3 For the feature value $x_n$ related to the variation information, the prior probability of each feature value class was calculated based on the selected third training set, and the number of occurrences of each class of data in the third training set was estimated. Taking the feature value x1 when the variation type was c1 as an example, the prior probability thereof was:

$$P(x_i = x_1 | c_k = c_1) = \frac{\text{the total amount of data for } x_i = x_1}{\text{the total amount of data}}.$$

4.1.4 Training. The prior probability $P(c_k)$ and class conditional probability were calculated based on a third training set of 674 variation sites. The samples in the third training set were divided into $K$ classes, e.g., $K = 3$, the class was $c_1, c_2, c_3$. For class $c_k$ (k = 1, 2, 3), presuming that $c_1 = 0$ represents non-variation sites, $c_2 = 1$ represents homozygous variation sites, $c_3 = 2$ represents heterozygous variation sites, Bayes formula was used to estimate conditional probability $P(c_k | x)$ when a training data $x$ was given, i.e.,

$$P(c_k|x) = \frac{P(c_k) \prod_{i=1}^{n} P(x_i|c_k)}{P(x)}.$$

Taking variation chr2-223086092-G-A as an example, the result of training calculation was: P($c_1$|x) = 0.00190984495430601 ; P($c_2$|x) = 3.63E - 07 ; P($c_3$|x) = 0.998089791607098.

4.1.5 Evaluation of model results and determination of quality control threshold

The performance of the above-mentioned machine learning model obtained by training was evaluated by the third test set, and the evaluation indexes may include accuracy rate, precision rate, and recall rate. 169 variation data selected above were used as the third test set. A variation type corresponding to the maximum prediction probability greater than a preset threshold was selected as the variation type prediction result of the variation site. The preset threshold was set to 0.8. The detailed test results are listed in Table 11. It should be noted that the preset threshold of the variation type prediction model can be adjusted based on the actual situation.

[Table 11]

|  | Precision rate | Recall rate | Accuracy rate |
|---|---|---|---|
| No variation | 95.56% | 89.58% | 95.86% |
| Homozygous variation | 88.24% | 100.00% | 96.45% |
| Heterozygous variation | 100.00% | 93.75% | 98.22% |

4.2 Application

4.2.1 The constructed variation type prediction model predicted the probability $P(c_k|x)$ that the variation site to be detected belongs to each class, wherein the variation site to be detected can be a variation site filtered from the above-mentioned first variation site and determined to be reliable by the first phenotype prediction model and the second phenotype prediction model without manual review.

4.2.2 The variation type prediction result was specifically determined by selecting the variation type corresponding to the maximum prediction probability and having a prediction result greater than a preset threshold of 0.8 as the variation type predication result of the variation site. For example, for the site with the variation interpretation result as pathogenic variation, when the variation type prediction result is a homozygous variation or heterozygous variation, it is determined that the variation site is reliable, and no experimental verification is required; and when the variation type prediction result is a no variation type, it is determined that the variation site is unreliable, and experimental verification is required.

Evaluation of overall performance of the present disclosure

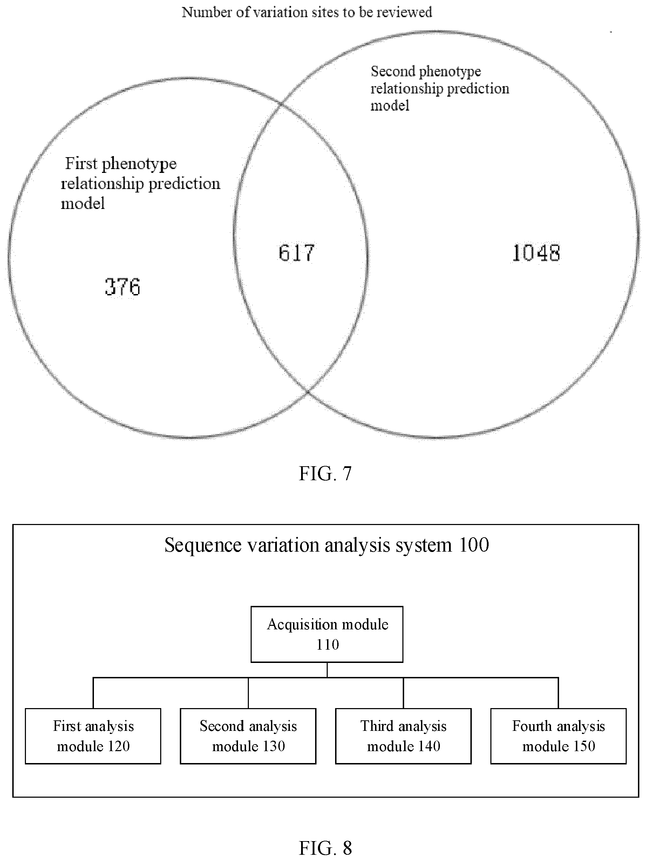

7,937 variation sites of 599 samples were selected as the sequence variation data to be analyzed. Interpretation results were obtained by means of manual interpretation of the variation data, and the results of the manual interpretation were compared with the results obtained according to various aspects of the present disclosure. Through the first variation sites filtering, 690 first variation sites were filtered out, thereby reducing the review workload by 8.69%. After determination of complex variation, 34 complex variation sites were determined, which required a combined interpretation for remedying the defects of variation detection tools. Based on the analysis results of the first phenotype relationship prediction model and the second phenotype relationship prediction model (as illustrated in FIG. 7), compared with other simple pathogenicity prediction models, the combination of the two models can obtain 2,041 variation sites inconsistent with the two models in the interpretation results, and it was determined that the interpretation results of 5,206 variation sites of 7,247 variation sites were reliable and unnecessary to be reviewed, thereby reducing the review workload by 71.84% and obtaining a more comprehensive manual review amount. Through the analysis of the variation type prediction model, it can be further determined that 61 of 76 pathogenic variations were not required to be verified, reducing 80.26% of the workload of variance verification.

In summary, the sequence variation analysis method according to the embodiments of the present disclosure can quickly, accurately, and comprehensively determine the variation to be filtered, reviewed, and verified. Only the interpretation results determined to be unreliable are required to be reviewed and maintained, and only the pathogenic variation determined to be unreliable is required to be verified. Therefore, it can significantly reduce the labor cost, improve the review and verification efficiency of gene variation sites, shorten the issuing time of gene detection reports, and improve the accuracy of interpretation of gene detection reports, thereby optimizing the whole interpretation and analysis process.

Based on the above-described sequence variation analysis method, the present disclosure proposes a computer-readable storage medium.

In this embodiment, a computer-readable storage medium has a computer program stored thereon. The computer program, when executed by a processor, implements the sequence variation analysis method described above.

FIG. 8 is a structure block diagram of a sequence variation analysis system according to an embodiment of the present disclosure.

The sequence variation analysis system 100 includes an acquisition module 110 and a first analysis module 120.

The acquisition module 110 is configured to acquire sequence variation data to be analyzed. The first analysis module 120 is configured to: obtain a first variation feature set and a second variation feature set by performing feature extraction on the sequence variation data to be analyzed; input the first variation feature set into a trained first phenotype relationship prediction model, to obtain a first phenotype relationship prediction result, and input the second variation feature set into a trained second phenotype relationship prediction model, to obtain a second phenotype relationship prediction result; and obtain a third phenotype relationship prediction result by taking a union of the first phenotype relationship prediction result and the second phenotype relationship prediction result.

In an embodiment of the present disclosure, the sequence variation analysis system 100 may further include a second analysis module 130.

The second analysis module 130 is configured to filter a first variation site in the sequence variation data to be analyzed. The first variation site is located in a simple repeat region of a reference sequence. The first variation site has a weak correlation with a phenotypic alteration. The first variation site is a non-single base variation.

In an embodiment of the present disclosure, the sequence variation analysis system 100 may further include a third analysis module 140.

The third analysis module 140 is configured to: acquire an amino acid level annotation result of the sequence variation data to be analyzed; and judge, based on the amino acid level annotation result, whether a complex variation exists in the sequence variation data to be analyzed

In an embodiment of the present disclosure, the sequence variation analysis system 100 may further include a fourth analysis module 150.

The fourth analysis module 150 is configured to: obtain a third variation feature set by performing feature extraction on the sequence variation data to be analyzed; input the third variation feature set into a trained variation type prediction model, to obtain a prediction probability of each variation type to which each variation site in the sequence variation data to be analyzed belongs; and determine a variation type prediction result of the corresponding variation site based on the prediction probability.

It should be noted that other embodiments of the sequence variation analysis system 100 of embodiments of the present disclosure may be referred to the embodiments of the sequence variation analysis methods of the above-described embodiments of the present disclosure.

In summary, the sequence variation analysis system according to the embodiments of the present disclosure can quickly, accurately, and comprehensively determine the variation to be filtered, reviewed, and verified. Only the interpretation results determined to be unreliable are required to be reviewed and maintained, and only the pathogenic variation determined to be unreliable is required to be verified. Therefore, it can significantly reduce the labor cost, improve the review and verification efficiency of gene variation sites, shorten the issuing time of gene detection reports, and improve the accuracy of interpretation of gene detection reports, thereby optimizing the whole interpretation and analysis process.

The above embodiments are provided only to illustrate, rather than limiting, the technical solution of the present disclosure. Although the present disclosure is described in detail with reference to the foregoing embodiments, those skilled in the art can appreciate that the technical solutions disclosed in the above-mentioned embodiments can be modified, or some of the technical features thereof can be replaced by equivalents. Such modifications and substitutions without departing from the spirit and scope of the embodiments of the present disclosure shall be included within the scope of the present disclosure.

**Claims**

1. A sequence variation analysis method, comprising:

    acquiring sequence variation data to be analyzed;
    obtaining a first variation feature set and a second variation feature set by performing feature extraction on the sequence variation data to be analyzed;

inputting the first variation feature set into a trained first phenotype relationship prediction model, to obtain a first phenotype relationship prediction result, and inputting the second variation feature set into a trained second phenotype relationship prediction model, to obtain a second phenotype relationship prediction result; and obtaining a third phenotype relationship prediction result by taking a union of the first phenotype relationship prediction result and the second phenotype relationship prediction result.

2. The sequence variation analysis method of claim 1, further comprising, subsequent to said obtaining the third phenotype relationship prediction result:

comparing the third phenotype relationship prediction result with a corresponding variation interpretation result;
determining, in response to that the third phenotype relationship prediction result is consistent with the variation interpretation result, the variation interpretation result to be reliable; and
determining, in response to that the third phenotype relationship prediction result is inconsistent with the variation interpretation result, the variation interpretation result to be unreliable.

3. The sequence variation analysis method of claim 1, further comprising:
filtering a first variation site in the sequence variation data to be analyzed, wherein:
the first variation site is located in a simple repeat region of a reference sequence, the first variation site has a weak correlation with a phenotypic alteration, and the first variation site is a non-single base variation.

4. The sequence variation analysis method of claim 3, wherein the weak correlation with the phenotypic alteration means that the variation is located in a non-coding region or an intron region and an allele frequency of the variation is greater than 0.05.

5. The sequence variation analysis method of claim 3, wherein the first variation site further satisfies any one of the following conditions:

condition 1: the total number of detections being greater than a first preset value, and the number of low-quality detections being greater than a second preset value; and
condition 2: located within a third preset value of upstream and downstream of a physical position of a reference sequence of a variation site satisfying condition 1.

6. The sequence variation analysis method of claim 1, further comprising:

acquiring an amino acid level annotation result of the sequence variation data to be analyzed; and
judging, based on the amino acid level annotation result, whether a complex variation exists in the sequence variation data to be analyzed.

7. The sequence variation analysis method of claim 6, wherein the variation sites where the complex variation exists satisfy the following conditions:

at least two of variation site reference sequence coordinates resulting in an amino acid change being overlapped; or
at least two of the variation site reference sequence coordinates resulting in the amino acid change being adjacent and affecting the same encoded amino acid.

8. The sequence variation analysis method of claim 1, further comprising:

obtaining a third variation feature set by performing feature extraction on the sequence variation data to be analyzed;
inputting the third variation feature set into a trained variation type prediction model, to obtain a prediction probability of each variation type to which each variation site in the sequence variation data to be analyzed belongs; and
determining a variation type prediction result of the corresponding variation site based on the prediction probability.

9. The sequence variation analysis method of claim 8, wherein said determining the variation type prediction result of the corresponding variation site based on the prediction probability comprises:
selecting a variation type corresponding to the maximum prediction probability greater than a preset threshold as the variation type prediction result of the variation site.

10. The sequence variation analysis method of claim 9, wherein said determining the variation type prediction result of the corresponding variation site further comprises:

determining, when the variation type prediction result only comprises a reference sequence genotype, the variation site to be unreliable; and
determining, when the variation type prediction result comprises at least one non-reference sequence genotype, the variation site to be reliable.

11. The sequence variation analysis method of claim 8, wherein:

the third variation feature value comprises variation support data and allele frequency; and
the variation type comprises homozygous variation, heterozygous variation, and no variation.

12. The sequence variation analysis method of claim 1, wherein:

each first variation feature value in the first variation feature set is a published phenotype relationship determination result of a variation site; and
each second variation feature value in the second variation feature set is allele frequency data of the variation site and functional prediction data.

13. The sequence variation analysis method of claim 12, wherein the functional prediction data in the second variation feature value comprises at least one of protein conservation prediction data, nucleic acid conservation prediction data, cleavage hazard prediction data, and variation site harmful degree prediction data.

14. A computer-readable storage medium, having a computer program stored thereon, wherein the computer program, when being executed by a processor, implements the sequence variation analysis method of any one of claims 1 to 13.

15. A sequence variation analysis system, comprising:

an acquisition module configured to acquire sequence variation data to be analyzed; and
a first analysis module configured to:

obtain a first variation feature set and a second variation feature set by performing feature extraction on the sequence variation data to be analyzed;
input the first variation feature set into a trained first phenotype relationship prediction model, to obtain a first phenotype relationship prediction result, and input the second variation feature set into a trained second phenotype relationship prediction model, to obtain a second phenotype relationship prediction result; and
obtain a third phenotype relationship prediction result by taking a union of the first phenotype relationship prediction result and the second phenotype relationship prediction result.

16. The sequence variation analysis system of claim 15, further comprising a second analysis module configured to filter a first variation site in the sequence variation data to be analyzed, wherein:
the first variation site is located in a simple repeat region of a reference sequence, the first variation site has a weak correlation with a phenotypic alteration, and the first variation site is a non-single base variation.

17. The sequence variation analysis system of claim 15, further comprising a third analysis module configured to:

acquire an amino acid level annotation result of the sequence variation data to be analyzed; and
judge, based on the amino acid level annotation result, whether a complex variation exists in the sequence variation data to be analyzed.

18. The sequence variation analysis system of claim 15, further comprising a fourth analysis module configured to:

obtain a third variation feature set by performing feature extraction on the sequence variation data to be analyzed;

input the third variation feature set into a trained variation type prediction model, to obtain a prediction probability of each variation type to which each variation site in the sequence variation data to be analyzed belongs; and determine a variation type prediction result of the corresponding variation site based on the prediction probability.

| Acquiring sequence variation data to be analyzed | S1 |

| Obtaining a first variation feature set and a second variation feature set by performing feature extraction on the sequence variation data to be analyzed | S2 |

| Inputting the first variation feature set into a trained first phenotype relationship prediction model, to obtain a first phenotype relationship prediction result, and inputting the second variation feature set into a trained second phenotype relationship prediction model, to obtain a second phenotype relationship prediction result | S3 |

| Obtaining a third phenotype relationship prediction result by taking a union of the first phenotype relationship prediction result and the second phenotype relationship prediction result | S4 |

FIG. 1

Model training

Model application

FIG. 2

Acquiring sequence variation data to be analyzed — S1

Filtering a first variation site in the sequence variation data to be analyzed, the first variation site being located in a simple repeat region of a reference sequence, having a weak correlation with a phenotypic alteration and being a non-single base variation

S5

Obtaining a first variation feature set and a second variation feature set by performing feature extraction on the sequence variation data to be analyzed — S2

Inputting the first variation feature set into a trained first phenotype relationship prediction model, to obtain a first phenotype relationship prediction result, and inputting the second variation feature set into a trained second phenotype relationship prediction model, to obtain a second phenotype relationship prediction result — S3

Obtaining a third phenotype relationship prediction result by taking a union of the first phenotype relationship prediction result and the second phenotype relationship prediction result — S4

FIG. 3

Acquiring sequence variation data to be analyzed $\diagup$ S1

Acquiring an amino acid level annotation result of the sequence variation data to be analyzed $\diagup$ S6

Obtaining a first variation feature set and a second variation feature set by performing feature extraction on the sequence variation data to be analyzed $\diagup$ S2

Judging, based on the amino acid level annotation result, whether a complex variation exists in the sequence variation data to be analyzed $\diagup$ S7

Inputting the first variation feature set into a trained first phenotype relationship prediction model, to obtain a first phenotype relationship prediction result, and inputting the second variation feature set into a trained second phenotype relationship prediction model, to obtain a second phenotype relationship prediction result $\diagup$ S3

Obtaining a third phenotype relationship prediction result by taking a union of the first phenotype relationship prediction result and the second phenotype relationship prediction result $\diagup$ S4

FIG. 4

Acquiring sequence variation data to be analyzed — S1

Obtaining a third variation feature set by performing feature extraction on the sequence variation data to be analyzed — S8

Obtaining a first variation feature set and a second variation feature set by performing feature extraction on the sequence variation data to be analyzed — S2

Inputting the third variation feature set into a trained variation type prediction model, to obtain a prediction probability of each variation type to which each variation site in the sequence variation data to be analyzed belongs — S9

Inputting the first variation feature set into a trained first phenotype relationship prediction model, to obtain a first phenotype relationship prediction result, and inputting the second variation feature set into a trained second phenotype relationship prediction model, to obtain a second phenotype relationship prediction result — S3

Determining a variation type prediction result of the corresponding variation site based on the prediction probability — S10

Obtaining a third phenotype relationship prediction result by taking a union of the first phenotype relationship prediction result and the second phenotype relationship prediction result — S4

FIG. 5

FIG. 6

Number of variation sites to be reviewed

Second phenotype
relationship prediction
model

First phenotype
relationship prediction
model

617

1048

376

FIG. 7

Sequence variation analysis system 100

Acquisition module
110

| First analysis module 120 | Second analysis module 130 | Third analysis module 140 | Fourth analysis module 150 |

FIG. 8

| | INTERNATIONAL SEARCH REPORT | International application No. |
|---|---|---|
| | | **PCT/CN2021/131904** |

**A. CLASSIFICATION OF SUBJECT MATTER**

G16B 20/20(2019.01)i

According to International Patent Classification (IPC) or to both national classification and IPC

**B. FIELDS SEARCHED**

Minimum documentation searched (classification system followed by classification symbols)

G16B

Documentation searched other than minimum documentation to the extent that such documents are included in the fields searched

Electronic data base consulted during the international search (name of data base and, where practicable, search terms used)

CNPAT, WPI, EPODOC, CNKI, IEEE: 变异, 变体, 突变, 基因, 序列, 预测, 致病, 模型, 神经网络, 两个, 另一, 多个, 过滤, 氨基酸, genetic+, mutation, aberrance, sequence+, prognosis+, pathogenicity, model, neural network, two, another, multi+, filt +, amino acid

**C. DOCUMENTS CONSIDERED TO BE RELEVANT**

| Category* | Citation of document, with indication, where appropriate, of the relevant passages | Relevant to claim No. |
|---|---|---|
| X | US 2020342955 A1 (APOSTLE, INC.) 29 October 2020 (2020-10-29)<br>description, paragraphs [0008]-[0205], and figures 1-14 | 1-18 |
| A | CN 110832597 A (ILLUMINA INC.) 21 February 2020 (2020-02-21)<br>entire document | 1-18 |
| A | CN 111063392 A (GENETALKS BIOTECHNOLOGY (CHANGSHA) CO., LTD.) 24 April 2020 (2020-04-24)<br>entire document | 1-18 |
| A | CN 112687332 A (BEIJING BERRY GENOMICS CO., LTD.) 20 April 2021 (2021-04-20)<br>entire document | 1-18 |

☐ Further documents are listed in the continuation of Box C.  ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **13 July 2022** | **28 July 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2021/131904**

| Patent document cited in search report | | | Publication date (day/month/year) | Patent family member(s) | | | Publication date (day/month/year) |
|---|---|---|---|---|---|---|---|
| US | 2020342955 | A1 | 29 October 2020 | WO | 2019084559 | A1 | 02 May 2019 |
| | | | | EP | 3704640 | A1 | 09 September 2020 |
| CN | 110832597 | A | 21 February 2020 | KR | 20200011471 | A | 03 February 2020 |
| | | | | KR | 20220043252 | A | 05 April 2022 |
| | | | | JP | 2020525886 | A | 27 August 2020 |
| | | | | IL | 271092 | D0 | 30 January 2020 |
| | | | | CA | 3065784 | A1 | 17 October 2019 |
| | | | | AU | 2021257920 | A1 | 18 November 2021 |
| | | | | NL | 2020861 | B1 | 22 October 2019 |
| | | | | EP | 3622524 | A1 | 18 March 2020 |
| | | | | SG | 11201913009 R | A | 30 January 2020 |
| | | | | AU | 2019253021 | A1 | 19 December 2019 |
| | | | | JP | 2021170350 | A | 28 October 2021 |
| | | | | US | 2019318806 | A1 | 17 October 2019 |
| | | | | WO | 2019200338 | A1 | 17 October 2019 |
| | | | | CN | 110832597 | A | 21 February 2020 |
| CN | 111063392 | A | 24 April 2020 | None | | | |
| CN | 112687332 | A | 20 April 2021 | None | | | |

Form PCT/ISA/210 (patent family annex) (January 2015)